# EUROPEAN PATENT APPLICATION

(11) **EP 4 729 946 A1**
(43) Date of publication of application: **22.04.2026**
(21) Application number: 24383156.7
(22) Date of filing: 21.10.2024
(51) Int. Cl.: G01N 33/68

(54) **METHOD FOR PREDICTING MORTALITY RISK**

(71) Applicant: Fundación Pública Galega Instituto de Investigación Sanitaria de Santiago de Compostela, 15706 Santiago de Compostela A Coruña (ES); Servizo Galego de Saude, 15703 Santiago de Compostela (ES); Universidade de Santiago de Compostela, 15782 Santiago de Compostela (ES)
(72) Inventor: CARBALLO FERNÁNDEZ, Iago, 17806 Santiago de Compostela (ES); GONZÁLEZ QUINTELA, Arturo, 15706 Santiago de Compostela (ES); LADO BALEATO, Óscar, 15706 Santiago de Compostela (ES); GUDE SAMPEDRO, Francisco, 15706 Santiago de Compostela (ES)
(74) Representative: Hoffmann Eitle

(57) **Abstract**

The present invention refers to an *in vitro* method for predicting mortality risk, for instance all-cause mortality risk, cancer mortality risk or cardiovascular risk.

## Description

### FIELD OF THE INVENTION

The present invention refers to the medical field. Particularly, the present invention refers to an *in vitro* method for predicting mortality risk, preferably all-cause mortality risk, cancer mortality risk or cardiovascular risk.

### STATE OF THE ART

Glycans are simple or complex molecules that play a critical structural and metabolic role in a cell or organism. Glycation is a non-enzymatic reaction that leads to the binding of a glycan to a protein, lipid, or another glycan. However, glycosylation is a sophisticated biological process regulated by many genes, transcription factors and signalling pathways, that takes part of cotranslational and post-translational modifications. The comprehensive study of all glycan structures, generation, modification, degradation, regulation, and function of living beings is known as "glycomics".

At least half of all human proteins are glycoproteins and most of them are covalently linked to the amide nitrogen of an asparagine (Asn) that belongs to an Asn-X-Ser/Thr consensus sequence of a protein, where X can be any amino acid except Proline. Total plasma/serum N-glycome (TPSNG) encompasses all glycans linked to plasma/serum proteins through this type of bonding and their analysis show information about patterns of glycation (signatures) that are typical in different diseases. To the best of our knowledge, TSPNG has not been studied in cardiovascular nor in cancer nor all-cause mortality in depth.

On the other hand, mortality is the most classical primary endpoint in clinical research. All-cause mortality is influenced by a multitude of factors with a generally low relative weight, so it is usually more difficult to assess than specific deaths but helps to avoid bias. The leading causes of death globally are cardiovascular diseases and cancer.

There is an unmet medical need of finding strategies aimed at predicting mortality risk, for instance all-cause mortality risk, cancer mortality risk or cardiovascular risk. The present invention is focused on solving this problem and an innovative method for predicting mortality risk, particularly all-cause mortality risk, cancer mortality risk or cardiovascular risk, is herein provided.

### DESCRIPTION OF THE INVENTION

### Brief description of the invention

The present invention refers to an *in vitro* method for predicting mortality risk. Particularly, the aim of the present invention is to identify predictive biomarkers of all-cause, cancer, and cardiovascular mortality in a general adult population and explore its usefulness in the general adult population as a prognostic tool alone and in combination with age, sex, and other common risk variables.

The inventors of the present invention have explored the capacity of total serum N-glycome to predict mortality in a general adult population. The prospective cohort study was performed in a single municipality in Spain including a random sample of 1,516 adults. The participants were profiled for total serum N-glycome at baseline. Serum enzymatic N-glycan release was made on a robotic platform followed by HILIC-UPLC glycan separation. Their computerized medical records were checked at median follow up of 7.52 years to collect date and cause of all deaths. The N-glycan groups from total serum were used to develop prediction models of mortality.

The inventors of the present invention concluded that the total serum N-glycome peak 16, mainly composed by A2[3]BG1S[3]1 predisposed to all-cause mortality. The total serum N-glycome peak 22, mainly composed by FA2G2S[6]1, protected from all-cause mortality. The balance between both predicted over time all-cause mortality incidence (area under curve 0.810 [0.773-0.847]). Similar results were obtained for cancer mortality with N-glycome peaks 16, 17, 22, and 23 (0.786 [0.728-0.843]); and for cardiovascular mortality with N-glycome peaks 7 and 9 (0.747 [0.645-0.850]). Their predictive power had an independent and additive effect on classical prediction factors.

Consequently, the balances between specific total serum N-glycome peaks are independent predictors of all-cause, cancer, and cardiovascular mortality and may contribute significantly to improve prognostic tools. In conclusion, the ratio or interaction between total serum N-glycome peaks herein defined constitutes a predictive signature for mortality risk.

So, the first embodiment of the present invention refers to an *in vitro* method for predicting mortality risk, the method comprising the assessment of the abundance of at least a N-glycome peak selected from the list consisting of: N-glycome peak 16, N-glycome peak 22, N-glycome peak 17, N-glycome peak 23, N-glycome peak 7 and/or N-glycome peak 9 in a biological sample obtained from the subject.

The second embodiment of the present invention refers to the *in vitro* use of at least a N-glycome peak selected from the list consisting of: N-glycome peak 16, N-glycome peak 22, N-glycome peak 17, N-glycome peak 23, N-glycome peak 7 and/or N-glycome peak 9, or of a kit comprising reagents for assessing the abundance of at least a N-glycome peak selected from the list consisting of: N-glycome peak 16, N-glycome peak 22, N-glycome peak 17, N-glycome peak 23, N-glycome peak 7 and/or N-glycome peak 9, for predicting mortality risk.

In a preferred embodiment, an increased abundance of N-glycome peak 16, N-glycome peak 17, N-glycome peak 23, and/or N-glycome peak 7 (with respect to a pre-established threshold value) is an indication of increased mortality risk.

In a preferred embodiment, an increased abundance of N-glycome peak 16 (with respect to a pre-established threshold value) is an indication of all-cause mortality risk, an increased abundance of N-glycome peak 16, N-glycome peak 17 and/or N-glycome peak 23 (with respect to a pre-established threshold value) is an indication of increased cancer mortality risk, and/or an increased abundance of N-glycome peak 7 (with respect to a pre-established threshold value) is an indication of increased cardiovascular mortality risk.

In a preferred embodiment, increased abundance of N-glycome peak 22 and/or N-glycome peak 9 (with respect to a pre-established threshold value) is an indication of decreased mortality risk.

In a preferred embodiment, an increased abundance of N-glycome peak 22 (with respect to a pre-established threshold value) is an indication of decreased all-cause mortality risk or an indication of decreased cancer mortality risk, and/or wherein an increased abundance of N-glycome peak 9 (with respect to a pre-established threshold value) is an indication of decreased cardiovascular mortality risk.

In a preferred embodiment, the present invention refers to an *in vitro* method for predicting all-cause mortality risk, which comprises assessing the abundance of a combination of N-glycome peaks comprising: N-glycome peak 16 and N-glycome peak 22, wherein an increased abundance of N-glycome peak 16 (with respect to a pre-established threshold value) is an indication of increased all-cause mortality risk, and/or wherein an increased abundance of N-glycome peak 22 (with respect to a pre-established threshold value) is an indication of decreased all-cause mortality risk.

In a preferred embodiment, the present invention refers to an *in vitro* method for predicting cancer mortality risk, which comprises assessing the abundance of a combination of N-glycome peaks comprising: N-glycome peak 16, N-glycome peak 17, N-glycome peak 23 and N-glycome peak 22, wherein an increased abundance of N-glycome peak 16, N-glycome peak 17 and/or N-glycome peak 23 (with respect to a pre-established threshold value) is an indication of increased cancer mortality risk, and/or wherein an increased abundance of N-glycome peak 22 (with respect to a pre-established threshold value) is an indication of decreased cancer mortality risk.

In a preferred embodiment, the present invention refers to an *in vitro* method for predicting cardiovascular mortality risk, which comprises assessing the abundance of a combination of N-glycome peaks comprising: N-glycome peak 7 and N-glycome peak 9, wherein an increased abundance of N-glycome peak 7 (with respect to a pre-established threshold value) is an indication of increased cardiovascular mortality risk, and/or wherein an increased abundance of N-glycome peak 9 (with respect to a pre-established threshold value) is an indication of decreased cardiovascular mortality risk.

In a preferred embodiment, the biological sample is selected from blood, plasma or serum.

In a preferred embodiment, the N-glycome peak 16 comprises the glycans: A2[3]BG1S[3]1, A2[3]BG1S[6]1, M7 D1, FA2[6]G1S[3]1, FA2[6]G1S[6]1, M4A1G1S[3]1, M4A1G1S[6]1; the N-glycome peak 22 comprises the glycans: FA2G2S[6]1, FA2G2S[3]1, M8 D1 D3; the N-glycome peak 17 comprises the glycans: FA2[3]G1S[6]1, FA2[3]G1S[3]1, F A2[6]BG 1 S[6] 1, FA2[6]BG1S[3]1; the N-glycome peak 23 comprises the glycans: FA2BG2S[3]1, FA2BG2S[6]1, the N-glycome peak 7 comprise the glycan: A2[6]BG1 and the N-glycome peak 9 comprise the glycan: FA2[3]G1.

In a preferred embodiment, the N-glycans are separated and quantified using a quantitative analytical technique.

In a preferred embodiment, the abundance of N-glycome peaks are assessed by chromatography, preferably by ultra-performance liquid chromatography (UPLC), capillary electrophoresis (CE), MALDI-TOF mass spectrometry, liquid chromatography coupled with mass spectrometry (LC-MS).

In a preferred embodiment, the abundance of N-glycome peaks are assessed by ultra-performance liquid chromatography (UPLC).

The present invention also refers to:

A method for treating patient suffering from increased mortality risk, particularly all-cause mortality risk, cancer mortality risk or cardiovascular risk, wherein the method comprises a previous step of predicting the risk of suffering from increased mortality risk, particularly all-cause mortality risk, cancer mortality risk or cardiovascular mortality risk by performing the above explained method. The therapy may comprise any treatment known in the prior art to treat or prevent cancer or cardiovascular diseases.

Regarding cancer treatments generally fall into a few main categories. Surgery involves physically removing the tumor from the body. Chemotherapy uses drugs to kill cancer cells or stop them from growing. Radiation therapy uses high-energy rays to target and destroy cancer cells. Immunotherapy boosts the body's immune system to fight cancer, while targeted therapy uses drugs to attack specific cancer cells without harming normal cells. Hormone therapy is used for cancers that rely on hormones to grow. These treatments can be used alone or in combination, depending on the type and stage of cancer.

Cardiovascular disease treatments generally fall into three main categories: medical, surgical, and lifestyle-based therapies. Medical treatments involve the use of medications to manage blood pressure, lower cholesterol, prevent blood clots, and improve blood flow. Common drugs include antihypertensives, statins, blood thinners, and beta-blockers. Surgical and interventional procedures are used in more advanced or complicated cases. These include angioplasty, where a balloon is used to open blocked arteries, stent placement (a device that keeps arteries open), or coronary artery bypass surgery, which reroutes blood around a blocked artery. Lifestyle-based therapies are essential in managing and preventing cardiovascular disease. These involve adopting a heart-healthy diet, reducing salt and fat intake, losing weight, exercising regularly, and quitting smoking. Cardiac rehabilitation may also be recommended after a heart event or surgery to help patients regain heart function. These treatments are often used in combination to optimize outcomes and prevent future complications.

In a preferred embodiment, the present invention is a computer-implemented invention, wherein a processing unit (hardware) and a software are configured to: a) Receive N-glycome peak values, b) process the values received for finding substantial variations or deviations, and c) provide an output through a terminal display of the variation or deviation of the value, wherein the variation or deviation of the value indicates the mortality risk, particularly all-cause mortality risk, cancer mortality risk or cardiovascular mortality risk.

For the purpose of the present invention the following terms and abbreviations are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- "N-glycome" refers to the complete set of N-linked glycans (sugar chains) attached to proteins within a cell, tissue, or organism. N-glycans are a type of carbohydrate chain that is attached to the nitrogen atom (N) in the side chain of an asparagine amino acid residue in a protein. This process, known as N-glycosylation, is critical for the proper folding, stability, and function of many proteins. The N-glycome is important in various biological processes, including cell-cell communication, immune response, and protein trafficking. Alterations in the N-glycome can be associated with diseases, making it a significant area of study in glycomics and biomedicine. N-glycans are herein identified using Oxford type or Oxford method nomenclature such as it is identified by this reference [Harvey DJ, Merry AH, Royle L, Campbell MP, Dwek RA, Rudd PM. Proposal for a standard system for drawing structural diagrams of N- and O-linked carbohydrates and related compounds. Proteomics. 2009;9(15):3796-801. doi: 10.1002/pmic.200900096*.* Erratum in: Proteomics. 2009;9(21):5002*].*
- According to the present invention, a reference value can be a "pre-established threshold value" or a "cut-off' value. Typically, a "threshold value" or "cut-off value" can be determined experimentally, empirically, or theoretically. Thus, for instance, the subject is likely to suffer from a higher mortality risk when an increased abundance of the claimed N-glycome is identified, as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Brief description of the figures

**Figure 1****.** Effect of GP16 and GP22 on the incidence of all-cause death using a balance [B(GP16,GP22)]. Abbreviations: B, balance; GP, glycome peak.
**Figure 2****.** Cumulative incidence of all-cause death stratified by B(GP16,GP22). Abbreviations: B, balance; GP, glycome peak.
**Figure 3****.** Cumulative incidence of cancer death stratified by B([GP16,GP17,GP23]GP22) **(panel a)**; cumulative incidence of cardiovascular death stratified by B[GP7,GP9) **(panel b)**. Abbreviations: B, balance; GP, glycome peak.

### Detailed description of the invention

The present invention is ilustrated by means of the Examples set below without the intention of limiting its scope of protection.

### Example 1. Material and methods

### Example 1.1. Study design and data sources

The A-Estrada Glycation and Inflammation study (AEGIS) is a prospective cohort study developed in A-Estrada, a municipality in Northwestern Spain. An outline of the study is available at www.clinicaltrials.gov (code NCT01796184). A-Estrada had an adult population (age >18 years) of 18,474 when the study began in 2012. An age-stratified random sample of 3500 adult individuals was drawn from Spain's National Health System Registry, which covers more than 95% of the population. From the initial 3500 individuals, 2230 could be assessed for eligibility and displayed no exclusion criteria (no health care, moved away, died, no response to communication attempts); of these, 1516 individuals agreed to participate (overall participation rate, 68%). Participation was higher among women than men (71% vs. 65%). There were no significant differences in terms of age or residence (rural vs. urban) between participants and non-participants.

From November 2012 through March 2015, all participants were successively contacted and asked to attend the Primary Care Center for evaluation through a clinical interview that included a structured questionnaire, physical examination, and fasting venous blood sampling (n=1516, 44.7% men, aged 18-91 years, median 52 years). All participants were again evaluated by a physician who checked computerized medical records until February 2023, which included all information about primary and hospital medical care provided by the Spanish National Health System. Participants with diagnosis of death at medical records were classified as deceased. The date and cause of death were also collected.

### Example 1.2. Ethical issues

All participants provided written informed consent. The general survey was approved by the Galician Regional Ethics Committee (code 2010-315) and conformed to the current Helsinki Declaration.

### Example 1.3. Assessment of overweight and obesity

The body mass index (BMI) was calculated as the weight (in kilograms) divided by the square of height (in meters). Participants were classified according to body mass index as normal weight (<25 kg/m2), overweight (25 to 30 kg/m2), or obese (>30 kg/m2).

### Example 1.4. Assessment of metabolic abnormalities

Participants were considered as having metabolic syndrome if they had at least 3 of the following Adult Treatment Panel III criteria [*Expert panel on detection, evaluation and treatment of high blood cholesterol in adults. Executive summary of third report of the National Cholesterol Education Program (NCEP) expert panel on detection, evaluation, and treatment of high blood cholesterol in adults (Adult Treatment Panel III).* JAMA 2001;285:2486-97*]:* 1) abdominal obesity; 2) hypertriglyceridemia; 3) low levels of high-density lipoprotein cholesterol; 4) increased blood pressure; and 5) hyperglycemia.

### Example 1.5. Assessment of smoking and alcohol consumption

Consumers of at least 1 cigarette per day were classified as smokers. Individuals who had quit smoking during the preceding year were still considered smokers.

Alcohol consumption was evaluated in standard drinking units by summing the number of glasses of wine (1 unit, ~10 g), bottles of beer (1 unit, ~10 g), and spirits (2 units, ~20 g) regularly consumed per week. Individuals with an alcohol consumption of 1-13 units/week were considered as light drinkers, those with 14-27 units/week as moderate drinkers, and those with ≥28 units/week as heavy drinkers.

### Example 1.6. Assessment of physical activity

All study participants completed the short version of International Physical Activity Questionnaire (freely available at https://sites.google.com/site/theipaq/home), which has been validated in Spain. The questionnaire allows for the calculation of metabolic equivalents of various tasks and for stratification of habitual physical activity as low, moderate, or high.

### Example 1.7. Assessment of glomerular filtration rate

The glomerular filtration rate (GFR) was estimated by the 4-variable Modification of Diet in Renal Disease (MDRD-4) equation using standardized serum creatinine values measured with isotope dilution mass spectrometry (IDMS)-traceable assays (MDRD-4-IDMS equation): estimated GFR = 175 × (standardized creatinine)^{-1.154} × (age)^{-0.203} × 1.212 [if black] × 0.742 [if female].

### Example 1.8. Determination of fasting plasma glucose

Glucose levels were determined in fresh serum samples from fasting participants by the glucose oxidase method in an ADVIA 2400 Clinical Chemistry System (Siemens, Germany).

### Example 1.9. Determination of glycated hemoglobin

Glycated hemoglobin (HbA1c) levels were determined in fresh serum samples by highperformance liquid chromatography in an ADAMS Aic HA-8160 analyzer (ARKRAY, Japan); all HbAlc values were converted to Diabetes Control and Complications Trial (DCCT)-aligned values.

### Example 1.10. Determination of cholesterol

Cholesterol levels were determined in fresh serum samples from fasting participants by the enzymatic method in an ADVIA 2400 Clinical Chemistry System (Siemens, Germany).

### Example 1.11. Low-density lipoprotein cholesterol and high-density lipoprotein cholesterol assays

Low-density (LDL) lipoprotein cholesterol, and high-density lipoprotein (HDL) cholesterol levels were determined in fresh serum samples from fasting participants by the elimination/catalase method in an ADVIA 2400 Clinical Chemistry System (Siemens, Germany).

### Example 1.12. Aspartate aminotransferase assay

Aspartate aminotransferase levels were determined in fresh serum samples from fasting participants by the IFFC (International Federation of Clinical Chemistry) modified method in an ADVIA 2400 Clinical Chemistry System (Siemens, Germany).

### Example 1.13. C-reactive protein assay

Wide-range C-reactive protein (CRP) concentrations were measured in fresh serum samples using commercial latex-enhanced immunoturbidimetry in an ADVIA 2400 Clinical Chemistry System (Siemens, Germany).

### Example 1.14. Erythrocyte Sedimentation Rate assay

Erythrocyte Sedimentation Rate (ESR) was measured in blood drawn in vacuum tubes containing K3EDTA (Becton Dickinson, USA) employing an automated TEST-1 device (Alifax, Italy).

### Example 1.15. Interleukin-6 assay

Interleukin-6 concentrations were measured in fresh serum samples using a commercial chemiluminescent immunoassay in an IMMULITE 2000 System (Siemens, Germany).

### Example 1.16. Tumor necrosis factor-alpha assay

Tumor necrosis factor (TNF)-alpha concentrations were measured in fresh serum samples using a commercial chemiluminescent immunoassay in an IMMULITE 2000 System (Siemens, Germany).

### Example 1.17. Serum N-glycan analyses

N-glycans were profiled by a modified high-throughput automated method from 5 µL of serum samples previously stored at -80°C for further use. Briefly, the samples were denatured and N-glycans were enzymatically released from the protein backbone via peptide:N-glycosidase F. The glycans were then immobilized on solid supported hydrazide beads, and excess reagents were removed by centrifuge filtration. The glycans were released from the solid support and labeled with fluorophore 2-aminobenzamide.

Hydrophilic interaction chromatography (HILIC) ultra-performance liquid chromatography (UPLC) was performed, assigning glucose unit values from retention times. The chromatograms were all separated in the same manner into 46 peaks, and the amount of glycans in each peak was expressed as a percentage of the total integrated area. Glycan structures were annotated using the symbol nomenclature for glycans and DrawGlycan-SNFG, with the assistance of GlycoStore.org.

Groups of glycome peaks (GPs) were defined from their common features:
- Sialylation: S0 (neutral, GP1-15); S1 (monosialylated, GP16-23 + GP30); S2 (disialylated, GP24-29 + 31); S3 (trisialylated, GP32-40); and S4 (tetrasialylated, GP41-46).
- Galactosylation: G0 (agalactosylated, GP1-2 + GP4-5 + GP6/2 + GP12/2); G1 (monogalactosylated, GP3 + GP7-10 + GP12/2 + GP16-18 + GP21/2); G2 (digalactosylated, GP13-15 + GP19-20 + GP21/2 + GP22-28); G3 (trigalactosylated, GP29 + GP31-37); and G4 (tetragalactosylated, GP30 + GP38-46).
- Branching: A1 (monoantennary, GP1-3 + GP12/2 + GP21/2); A2 (biantennary, GP4-5 + GP6/2 + GP7-10 + GP12/2 + GP13-20 + GP21/2 + GP22-28); A3 (triantennary, GP29 + GP31-37); and A4 (tetra-antennary, GP30 + GP38-46).
- Oligomannose: GP6/2 + GP11.
- Fucosylation: Core-fucose (GP2 + GP5 + GP6/2 + GP8-10 + GP14-15 + GP17-18 + GP22-23 + GP27-28 + GP36 + GP44/2); and outer-arm fucose (GP37 + GP40 + GP41/3 + GP45 + GP46/3).

In addition, mass spectrometry-assisted glycan characterization was performed for 2 representative samples and a technical replicate. Otherwise, the major glycans were identified and assigned based on their glucose unit values cross-referenced in Glycobase, now migrated to Glycostore.

### Example 1.18. Statistical analysis

Mann-Whithney and Chi-squared tests were applied to check for differences in continuous and categorical variables, respectively, in the participant groups.

Survival time was defined from the date of enrolment in AEGIS to the date of exitus. Censored time was considered for participants who were alive at the end of the study or lost during follow-up. Cumulative incidence curve was obtained from the inverse of the Kaplan-Meier estimator, and differences in survival rates between patient groups were tested using the logrank test.

An automatic variable selection algorithm based on ridge regression techniques was used to establish main risk factors of death. Specifically, an elastic net regularization method was fitted where the choice of the optimum lambda parameter was based on the model C index obtained from 10 cross-validations. Selected variables, with P-values higher than 0.05 in a multivariate Cox regression model, were discarded from the final model. Moreover, non-linear effects of continuous variables were estimated through spline functions. Results were expressed as hazard ratios.

N-glycome data were analyzed following up to date compositional data analysis techniques. Exploratory data analysis and differences between participant groups were tested on clr tranformed values from the original data. The selbal algorithm was employed to determine the optimal combination of GPs for predicting death. Specifically, GPs entered the model as balances, which are normalised logarithmic ratios between two subsets of components from the original data. The variable selection algorithm initiated with a search to identify the two GPs whose balance was most strongly associated with the response variable (i.e., death), then the algorithm employed a forward selection process. At each step, the GP that most improved the area under the curve (AUC) of the model was added. This process continued until no further enhancement in classification accuracy could be achieved. The selected balances were included in a multivariate Cox regression model and their statistical significance were tested using a log-likelihood ratio test.

The AUC from the receiver operating characteristic (ROC) analysis were used to assess the GPs' predictive performance. The ROC curves and the AUC, with 95% confidence intervals, were calculated using the pROC R package. In addition, we estimated the time-dependent AUC to assess the predictive performance of the chosen balances along time. Statistical analyses were performed in R, using the packages' compositions and mgcv.

### Example 2. Results

### Example 2.1. All-cause mortality

During the follow-up, 130 out of 1516 participants died. Death incidence rate was 8.6% (95% CI 7.2-10.1) after a mean follow-up of 7.01 years by participant. Individuals were died were older, predominantly men, had a higher BMI, lower physical activity, higher proportion of metabolic syndrome, ex-smoking, and high or heavy alcohol consumption. Moreover, they showed higher values of glycemic (FPG, HbA1c) and inflammatory (C-reactive protein, TNF-alfa, ESR) markers, as well as lower glomerular filtration rate, lower levels of cholesterol, HDL cholesterol, and LDL cholesterol, and higher levels of aspartate aminotransferase. They also had higher prevalence of hypertension, diabetes mellitus, ischemic heart disease, heart failure, peripheral artery disease, stroke and cancer (**Table 1**).

**Table 1. Baseline characteristics of AEGIS participants stratified by alive or death status.**

| Variable | | AEGIS (n=1516) | Alive (n=1386) | Death (n=130) | P |
|---|---|---|---|---|---|
| Age (years)* | | 52 [39, 67] | 50 [38, 64] | 76 [66, 82] | <0.001 |
| Sex (female)^{†} | | 837 (55.2%) | 783 (56.5%) | 54 (41.5%) | 0.001 |
| BMI (kg/m²)* | | 27.8 [24.6, 31.4] | 27.6 [24.4, 31.2] | 29.8 [26.3, 34.1] | <0.001 |

| Smoking status^{†} | | | | | |
|---|---|---|---|---|---|
| | - Non | 825 (54.4%) | 757 (54.7%) | 67 (51.5%) | 0.000 |
| | - Ex | 395 (26.1%) | 344 (24.8%) | 51 (39.2%) | |
| | - Smoker | 296 (19.5%) | 284 (20.5%) | 12 (9.2%) | |

| Alcohol consumption^{†} | | | | | |
|---|---|---|---|---|---|
| | - Abstemious | 546 (36.0%) | 495 (35.7%) | 50 (38.5%) | 0.090 |
| | - Light drinker | 598 (39.5%) | 558 (40.3%) | 40 (30.8%) | |
| | - Moderate | 241 (15.9%) | 218 (15.7%) | 23 (17.7%) | |
| | - Heavy | 131 (8.6%) | 114 (8.2%) | 17 (13.1%) | |

| Physical activity^{†} | | | | | |
|---|---|---|---|---|---|
| | - Low | 596 (39.3%) | 528 (38.1%) | 68 (52.3%) | 0.002 |
| | - Medium | 552 (36.4%) | 508 (36.7%) | 44 (33.8%) | |
| | - High | 368 (24.2%) | 349 (25.2%) | 18 (13.8%) | |
| Metabolic syndrome^{†} | | 314 (20.7%) | 265 (19.1%) | 49 (37.7%) | <0.001 |
| Diabetes Mellitus^{†} | | 187 (12.3%) | 141 (10.2%) | 46 (35.4%) | <0.001 |
| FPG (mg/dL)* | | 89.0 [82.0, 100.0] | 88.0 [81.0, 98.0] | 100.5 [88.0, 117.0] | <0.001 |
| HbAlc (%)* | | 5.4 [5.2, 5.7] | 5.4 [5.2, 5.7] | 5.7 [5.4, 6.3] | <0.001 |
| Cholesterol (mg/dL)* | | 195.0 [169.0, 220.0] | 195.0 [170.0, 222.0] | 187.0 [164.2,213.0] | 0.011 |
| LDL chol (mg/dL)* | | 113.0 [94.0, 134.0] | 114.0 [94.0, 135.0] | 106.0 [86.0, 124.8] | 0.003 |
| HDL chol (mg/dL)* | | 57.0 [47.0, 69.0] | 58.0 [48.0,69.0] | 53.0 [45.0, 63.8] | 0.001 |
| ESR (mm/h)* | | 9.0 [5.0, 17.0] | 9.0 [5.0, 16.0] | 15.0 [7.8,28.0] | <0.001 |
| CRP (mg/dL)* | | 0.14 [0.04, 0.39] | 0.13 [0.04, 0.36] | 0.27 [0.10, 0.66] | <0.001 |
| TNF-alpha (pg/mL)* | | 7.4 [6.1, 9.0] | 7.2 [6.0, 8.7] | 8.3 [7.0, 9.5] | <0.001 |
| AST (UI/L)* | | 23.0 [19.0, 27.0 | 22.0 [19.0,27.0] | 24.0 [21.0,28.0] | 0.001 |
| GFR (mL/min)* | | 100.4 [86.9, 115.1] | 101.9 [88.3, 116.5] | 88.2 [70.3, 100.0] | <0.001 |
| Arterial hypertension^{†} | | 486 (32.1%) | 396 (28.6%) | 90 (69.2%) | <0.001 |
| Heart failure^{†} | | 26 (1.7%) | 11 (0.8%) | 15 (11.5%) | <0.001 |
| IHD^{†} | | 65 (4.3%) | 42 (3.0%) | 23 (17.7%) | <0.001 |
| PAD^{†} | | 27 (1.8%) | 15 (1.1%) | 12 (9.2%) | <0.001 |
| Stroke^{†} | | 35 (2.3%) | 22 (1.6%) | 13 (10.0%) | <0.001 |
| Cancer^{†} | | 71 (4.7%) | 53 (3.8%) | 18 (13.8%) | <0.001 |

| | | | | | |
|---|---|---|---|---|---|
| Abbreviations: AEGIS, A-Estrada Glycation and Inflammation Study; AST, aspartate aminotransferase; BMI, body mass index; CRP, C-reactive protein; ESR, erythrocyte sedimentation rate; FPG, fasting plasma glucose; GFR, glomerular filtration rate; HbA1c, glycated hemoglobin; HDL chol, high-density lipoprotein cholesterol; IHD, ischemic heart disease; LDL chol, low-density lipoprotein cholesterol; PAD, peripheral artery disease; TNF, tumor necrosis factor.*Median and [interquartile range], ^{†}absolute frequency and (percentage). | | | | | |

The participants who died showed a greater abundance of GP1, 2, 3, 5, 6, 7, 10, 16, 41, and 46 and lesser abundance of GP8, 9, 14, 17, 18, 20, 22, 24, 25, 26, 27, 29, 30, 31, 33, 34, 35, and 36 than those who were alive.

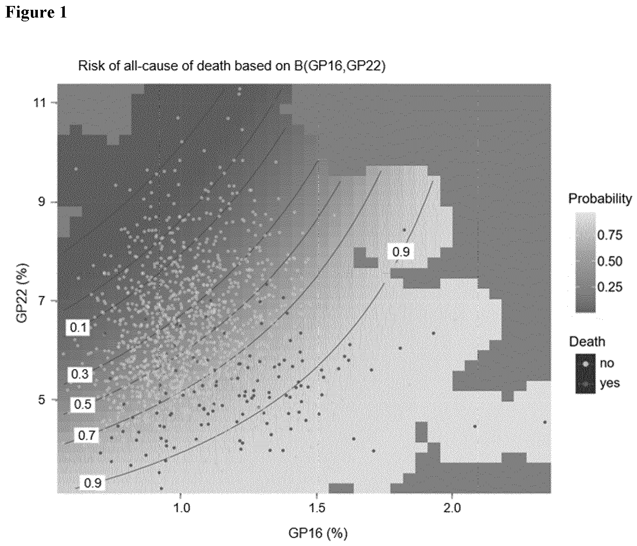

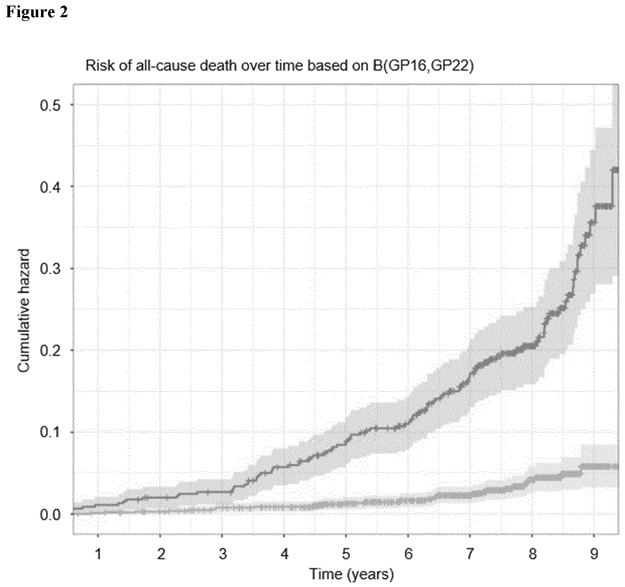

The *selbal* algorithm identified the balance (B): B(GP16,GP22) as the optimal balance for predicting all-cause mortality, with an AUC of 0.810 [95% CI 0.773-0.847]. Higher relative values of GP16 to GP22 showed a higher risk of death **(****Figure 1****)**. Those subjects with a value of the B(GP16,GP22) higher or equal to 1.15 displayed a cumulative incidence of death of 30.0% (95% CI 23.6-35.8) after 9 years, whereas those with lower values had a cumulative incidence of 5.6% (95% CI 3.2-8.0) **(****Figure 2****)**. The predictive accuracy of GP16 and 22 remained stable near 0.80 throughout the follow-up period. *N*-glycome is therefore a potential predictor of all-cause death, both in the short and long term.

The effect of B(GP16,GP22) on mortality, adjusted for common risk factors, was estimated using multivariate Cox regression models. The effect of B(GP16,GP22) on mortality was independent of age, sex, and other factors which were chosen using an elastic net variable selection algorithm **(Table 2).**

**Table 2. Effect of risk factors on all-cause mortality estimated using a multivariate Cox regression.**

| Predictor variables | B(GP16,GP22) | Age + sex | Age + sex + B(GP16,GP22) | Risk factors | Risk factors + B(GP16,GP22) |
|---|---|---|---|---|---|
| | HR (95% CI) | HR (95% CI) | HR (95% CI) | HR (95% CI) | HR (95% CI) |
| B(GP16,GP22) | 1.62 (1.49 - 1.76) | -- | 1.31 (1.19 - 1.45) | -- | 1.30 (1.17 - 1.43) |
| Age (years) | -- | 1.10 (1.09 - 1.12) | 1.09 (1.07 - 1.10) | 1.10 (1.08 -1.12) | 1.09 (1.07 - 1.11) |
| Sex (ref: female) | -- | 2.28 (1.60 - 3.25) | 2.51 (1.76 - 3.58) | 1.64 (1.04 - 2.60) | 1.86 (1.17 - 2.96) |
| BMI (kg/m²) | -- | -- | -- | 1.01 (0.97 - 1.05) | 1.01 (0.97 - 1.05) |
| Ex-smoker | -- | -- | -- | 1.57 (1.01 - 2.45) | 1.52 (0.96 - 2.41) |
| Smoker | -- | -- | -- | 1.56 (0.74 - 3.25) | 1.56 (0.75 - 3.25) |
| HDL chol (mmol/L) | -- | -- | -- | 0.99 (0.97 - 1.00) | 0.99 (0.97 - 1.00) |
| ESR (mm/h) | -- | -- | -- | 1.02 (1.01 - 1.03) | 1.02 (1.00 - 1.03) |
| AST (UI/L) | -- | -- | -- | 1.03 (1.02 - 1.05) | 1.03 (1.02 - 1.05) |
| Heart failure | -- | -- | -- | 3.06 (1.74 - 5.37) | 4.07 (2.31 - 7.16) |
| LogLik ratio test | -- | <0.001 | | <0.001 | |

| | | | | | |
|---|---|---|---|---|---|
| Risk factors of all-cause death were selected based on the elastic net regularization method. Abbreviations: AST, aspartate aminotransferase; B, balance; BMI, body mass index; ESR, erythrocyte sedimentation rate; GP, glycome peak; HDL chol, high-density lipoprotein cholesterol; HR, hazard ratio; ref, reference. | | | | | |

### Example 2.2. Cancer mortality

Deaths due to cancer were 43 out of 130 (33%). Cancer death incidence rate was 2.8% (95% CI 2.1-3.8) after a mean follow-up of 7.01 years by participant. Individuals who died of cancer were older, had a higher BMI, and higher proportion of ex-smoking and metabolic syndrome. Moreover, they showed higher values of glycemic (FPG, HbA1c) and inflammatory (C-reactive protein, TNF-alfa, ESR) markers, as well as lower glomerular filtration rate, lower levels of HDL cholesterol, and higher prevalence of hypertension, diabetes mellitus, ischemic heart disease, peripheral artery disease, and cancer **(Table 3).**

**Table 3. Baseline characteristics of AEGIS participants stratified by non-cancer death or cancer death status**

| Variable | | Non cancer death (n=1472) | Cancer death (n=43) | P |
|---|---|---|---|---|
| Age (years)* | | 52.0 [38.8, 66.0] | 660 [60.0,78.5] | <0.001 |
| Sex (female)^{†} | | 819 (55.6%) | 18 (41.9%) | 0.102 |
| BMI (kg/m²)* | | 27.7 [24.5, 31.3] | 30.0 [28.0, 34.6] | <0.001 |

| Smoking status^{†} | | | | |
|---|---|---|---|---|
| | - Non | 805 (54.7%) | 19 (44.2%) | 0.055 |
| | - Ex | 377 (25.6%) | 18 (41.9%) | |
| | - Smoker | 290 (19.7%) | 6 (14.0%) | |

| Alcohol consumption^{†} | | | | |
|---|---|---|---|---|
| | - Abstemious | 530 (36.0%) | 15 (34.9%) | 0.998 |
| | - Light drinker | 581 (39.5%) | 17 (39.5%) | |
| | - Moderate | 234 (15.9%) | 7 (16.3%) | |
| | - Heavy | 127 ( 8.6%) | 4 ( 9.3%) | |

| Physical activity^{†} | | | | |
|---|---|---|---|---|
| | - Low | 578 (39.3%) | 18 (41.9%) | 0.871 |
| | - Medium | 536 (36.4%) | 16 (37.2%) | |
| | - High | 358 (24.3%) | 9 (20.9%) | |
| Metabolic syndrome^{†} | | 295 (20.0%) | 19 (44.2%) | <0.001 |
| Diabetes Mellitus^{†} | | 174 (11.8%) | 13 (30.2%) | 0.001 |
| FPG (mg/dL)* | | 89.0 [82.0, 99.0] | 98.0 [87.0, 113.5] | <0.001 |
| HbAlc (%)* | | 5.4 [5.2, 5.7] | 5.7 [5.4, 6.0] | <0.001 |
| Cholesterol (mg/dL)* | | 195.0 [169.0, 220.0] | 195.0 [173.0,216.0] | 0.612 |
| LDL chol (mg/dL)* | | 114.0 [94.0, 135.0] | 107.0 [96.0, 123.5] | 0.138 |
| HDL chol (mg/dL)* | | 58.0 [47.0, 69.0] | 49.0 [40.5,63.5] | 0.013 |
| ESR (mm/h)* | | 9.0 [5.0, 16.0] | 16.0 [7.2,25.5] | 0.001 |
| CRP (mg/dL)* | | 0.1 [0.0, 0.4] | 0.3 [0.1, 0.8] | 0.002 |
| TNF-alpha (pg/mL)* | | 7.4 [6.1, 8.9] | 8.9 [6.9, 10.7] | 0.001 |
| AST (UI/L)* | | 23.0 [19.0, 27.0] | 23.0 [21.0, 28.0] | 0.101 |
| GFR (mL/min)* | | 100.6 [87.1, 115.1] | 960 [78.7, 107.2] | 0.047 |
| Arterial hypertension^{†} | | 461 (31.3%) | 25 (58.1%) | <0.001 |
| Heart failure^{†} | | 25 (1.7%) | 1 (2.3%) | 1.000 |
| IHD^{†} | | 59 (4.0%) | 6 (14.0%) | 0.005 |
| PAD^{†} | | 22 (1.5%) | 5 (11.6%) | <0.001 |
| Stroke^{†} | | 33 (2.2%) | 2 (4.7%) | 0.602 |
| Cancer^{†} | | 61 (4.1%) | 10 (23.3%) | <0.001 |

| | | | | |
|---|---|---|---|---|
| Abbreviations: AEGIS, A-Estrada Glycation and Inflammation Study; AST, aspartate aminotransferase; BMI, body mass index; CRP, C-reactive protein; ESR, erythrocyte sedimentation rate; FPG, fasting plasma glucose; GFR, glomerular filtration rate; HbA1c, glycated hemoglobin; HDL chol, high-density lipoprotein cholesterol; IHD, ischemic heart disease; LDL chol, low-density lipoprotein cholesterol; PAD, peripheral artery disease; TNF, tumor necrosis factor. *Median and [interquartile range], ^{†}absolute frequency and (percentage). | | | | |

The participants who died due to cancer showed a greater abundance of GP5, 6, 16, and 37 and lesser abundance of GP4, 14, 22, 24, 27, and 33 than those who did not die due to cancer.

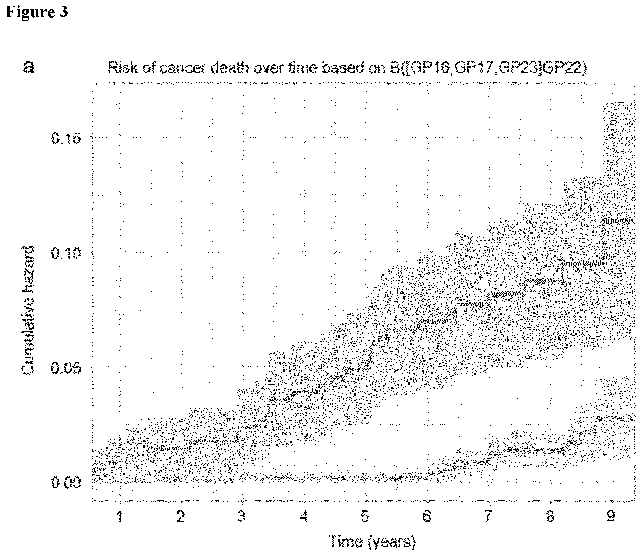

The *selbal* algorithm identified the B([GP16,GP17,GP23],GP22) as the optimal balance for predicting death due to cancer, with an AUC of 0.786 [95% CI 0.728-0.843]. Higher relative values of GP16, GP17, and GP23 to GP22 showed a higher risk of death by cancer. Those subjects with B([GP16,GP17,GP23],GP22) ≥1.12 displayed a cumulative incidence of death by cancer of 10.7% [95% CI 5.9-15.2] after 9 years, whereas those with lower values had a cumulative incidence of 2.7% [95% CI 0.9-4.4] **(****Figure 3a****)**. The predictive accuracy of GP16, 17, 23, and 22 decreased throughout the follow-up period. *N*-glycome is therefore a better potential predictor of cancer death in the short term.

The effect of B([GP16,GP17,GP23],GP22) on mortality, adjusted for common risk factors, was estimated using multivariate Cox regression models. The effect of B([GP16,GP17,GP23],GP22) on mortality was independent of age, sex, and other factors which were chosen using an elastic net variable selection algorithm **(Table 4)**.

**Table 4. Effect of risk factors on cancer mortality estimated using a multivariate Cox regression.**

| Predictor variables | B([GP16,GP17, GP23]GP22) | Age + sex | Age + sex + B([GP16,GP17, GP23]GP22) | Risk factors | Risk factors + B([GP16,GP17, GP23]GP22) |
|---|---|---|---|---|---|
| | HR (95% CI) | HR (95% CI) | HR (95% CI) | HR (95% CI) | HR (95% CI) |
| B([GP16,GP17, GP23]GP22) | 1.64(1.41 -1.91) | -- | 1.50 (1.25 - 1.80) | -- | 1.44 (1.17 - 1.78) |
| Age (years) | -- | 1.06 (1.03 - 1.08) | 1.03 (1.01 - 1.06) | 1.03 (1.01 - 1.06) | 1.02 (0.99 - 1.05) |
| Sex (ref: female) | -- | 2.10 (1.13 - 3.90) | 2.33 (1.25 - 4.34) | 1.67 (0.78 - 3.59) | 1.63 (0.75 - 3.53) |
| BMI (kg/m²) | -- | -- | -- | 1.04 (0.98 - 1.11) | 1.03 (0.96 - 1.09) |
| ESR (mm/h) | -- | -- | -- | 1.02 (1.00 - 1.04) | 1.01 (0.99 - 1.03) |
| Arterial hypertension | -- | -- | -- | 1.03 (1.01 - 1.05) | 1.03 (1.01 - 1.05) |
| Cancer | -- | -- | -- | 3.71 (1.74 - 7.90) | 4.04 (1.91 - 8.51) |
| LogLik ratio test | -- | <0.001 | | <0.001 | |

| | | | | | |
|---|---|---|---|---|---|
| *Risk factors of cancer death were selected based on the elastic net regularization method.* *Abbreviations: B, balance; BMI, body mass index; ESR, erythrocyte sedimentation rate; GP, glycome peak; HR, hazard ratio; ref, reference.* | | | | | |

### Example 2.3. Cardiovascular mortality

Deaths due to cardiovascular cause were 32 out of 130 (25%). Cardiovascular death incidence rate was 2.1% (95% CI 1.4-3.0) after a mean follow-up of 7.01 years by participant. Individuals who died of cardiovascular cause were older, predominantly men, had a higher BMI, lower physical activity, higher proportion of metabolic syndrome, and high or heavy alcohol consumption. Moreover, they showed higher values of glycemic (FPG, HbA1c) and inflammatory (C-reactive protein, TNF-alfa, ESR) markers, as well as lower glomerular filtration rate, and higher prevalence of hypertension, diabetes mellitus, ischemic heart disease, heart failure, peripheral artery disease, and stroke **(Table 5)**.

**Table 5. Baseline characteristics of AEGIS participants stratified by non-cardiovascular death or cardiovascular death status**

| Variable | | Non cardiovascular death (n=1484) | Cardiovascular death (n=32) | P |
|---|---|---|---|---|
| Age (years)* | | 52.0 [39.0, 66.0] | 76.5 [69.8, 82.0] | <0.001 |
| Sex (female)^{†} | | 826 (55.7%) | 11 (34.4%) | 0.026 |
| BMI (kg/m²)* | | 27.7 [24.6, 31.3] | 29.9 [27.3, 33.1] | 0.006 |

| Smoking status^{†} | | | | |
|---|---|---|---|---|
| | - Non | 806 (54.3%) | 18 (56.2%) | 0.271 |
| | - Ex | 384 (25.9%) | 11 (34.4%) | |
| | - Smoker | 293 (19.8%) | 3 (9.4%) | |

| Alcohol consumption^{†} | | | | |
|---|---|---|---|---|
| | - Abstemious | 539 (36.3%) | 6 (18.8%) | 0.040 |
| | - Light drinker | 586 (39.5%) | 12 (37.5%) | |
| | - Moderate | 233 (15.7%) | 8 (25.0%) | |
| | - Heavy | 125 (8.4%) | 6 (18.8%) | |

| Physical activity^{†} | | | | |
|---|---|---|---|---|
| | - Low | 575 (38.8%) | 21 (65.6%) | 0.005 |
| | - Medium | 543 (36.6%) | 9 (28.1%) | |
| | - High | 365 (24.6%) | 2 (6.2%) | |
| Metabolic syndrome^{†} | | 298 (20.1%) | 16 (50.0%) | <0.001 |
| Diabetes Mellitus^{†} | | 171 (11.5%) | 16 (50.0%) | <0.001 |
| FPG (mg/dL)* | | 88.0 [82.0, 99.0] | 109.5 [97.5, 136.5] | <0.001 |
| HbAlc (%)* | | 5.4 [5.2, 5.7] | 6.2 [5.8, 7.3] | <0.001 |
| Cholesterol (mg/dL)* | | 195.0 [169.0, 221.0] | 179.0 [165.2,212.0] | 0.151 |
| LDL chol (mg/dL)* | | 114.0 [94.0, 134.8] | 105.5 [85.0, 131.2] | 0.228 |
| HDL chol (mg/dL)* | | 58.0 [47.0, 69.0] | 54.5 [45.0, 63.2] | 0.197 |
| ESR (mm/h)* | | 9.0 [5.0, 16.0] | 19.0 [9.0, 30.0] | <0.001 |
| CRP (mg/dL)* | | 0.1 [0.0, 0.4] | 0.3 [0.1, 0.9] | 0.002 |
| TNF-alpha (pg/mL)* | | 7.4 [6.0, 8.9] | 9.9 [7.7, 11.0] | 0.001 |
| AST (UI/L)* | | 23.0 [19.0, 27.0] | 23.5 [20.0, 33.2] | 0.199 |
| GFR (mL/min)* | | 101.1 [87.1, 115.2] | 88.2 [73.2, 93.8] | <0.001 |
| Arterial hypertension^{†} | | 462 (31.2%) | 24 (75.0%) | <0.001 |
| Heart failure^{†} | | 20 (1.3%) | 6 (18.8%) | <0.001 |
| IHD^{†} | | 58 (3.9%) | 7 (21.9%) | <0.001 |
| PAD^{†} | | 22 (1.5%) | 5 (15.6%) | <0.001 |
| Stroke^{†} | | 31 (2.1%) | 4 (12.5%) | 0.001 |
| Cancer^{†} | | 66 (4.5%) | 5 (15.6%) | 0.011 |

| | | | | |
|---|---|---|---|---|
| *Abbreviations: AEGIS, A-Estrada Glycation and Inflammation Study; AST, aspartate aminotransferase; BMI, body mass index; CRP, C-reactive protein; ESR, erythrocyte sedimentation rate; FPG, fasting plasma glucose; GFR, glomerular filtration rate; HbA1c, glycated hemoglobin; HDL chol, high-density lipoprotein cholesterol; IHD, ischemic heart disease; LDL chol, low-density lipoprotein cholesterol; PAD, peripheral artery disease; TNF, tumor necrosis factor. *Median and (interquartile range], ^{†}absolutefrequency and (percentage).* | | | | |

The participants who died due to cardiovascular cause showed a greater abundance of GP 2, 3, 5, 6, 7, and 16 and lesser abundance of GP 14, 22, 25, and 27 than those who did not die due to cardiovascular cause.

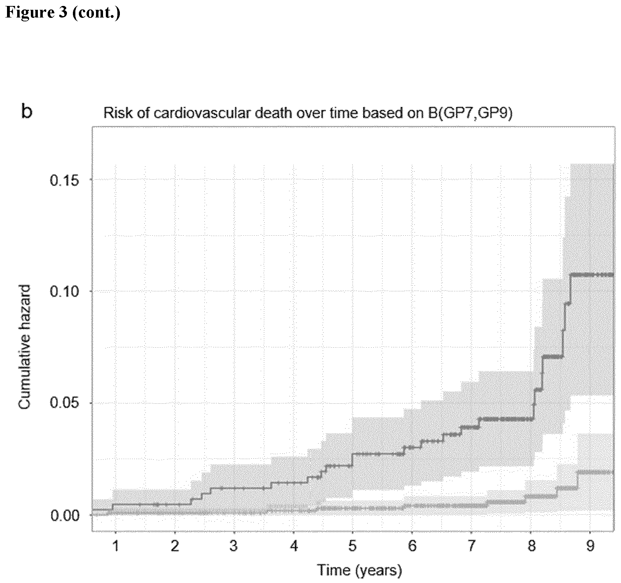

The *selbal* algorithm identified the B(GP7,GP9) as the optimal balance for predicting cardiovascular death, with an AUC of 0.747 [95% CI 0.645-0.850]. Higher relative values of GP7 to GP9 showed a higher risk of cardiovascular death. Those subjects with B(GP7,GP9) ≥1.53 displayed a cumulative incidence of cardiovascular death of 10.2% [95% CI 5.2-14.9] after 9 years, whereas those with lower values had a cumulative incidence of 1.89% [95% CI 1.9-3.5] **(****Figure 3b****)**. The time-dependent AUC for the B(GP7,GP9) showed high variability with broad confidence bands; however, it tended to decrease over time.

The effect of B(GP7,GP9) on mortality, adjusted for common risk factors, was estimated using multivariate Cox regression models. The effect of B(GP7,GP9) on mortality was independent of age, sex, and other factors which were chosen using an elastic net variable selection algorithm **(Table 6).**

**Table 6. Effect of risk factors on cardiovascular mortality estimated using a multivariate Cox regression.**

| Predictor variables | B(GP7,GP9) | Age + sex | Age + sex + B(GP7,GP9) | Risk factors | Risk factors + B(GP7,GP9) |
|---|---|---|---|---|---|
| | HR (95% CI) | HR (95% CI) | HR (95% CI) | HR (95% CI) | HR (95% CI) |
| B(GP7,GP9) | 1.33 (1.21 - 1.46) | -- | 1.16 (1.06 - 1.27) | -- | 1.22 (1.10 - 1.36) |
| Age (years) | -- | 1.14 (1.10 - 1.19) | 1.12 (1.08 - 1.16) | 1.13 (1.08 - 1.18) | 1.11 (1.06 - 1.16) |
| Sex (ref: female) | -- | 3.36 (1.59 - 7.09) | 2.87 (1.35 - 6.12) | 2.71 (1.13 - 6.51) | 2.55 (1.06 - 6.10) |
| BMI (kg/m2) | -- | -- | -- | 1.04 (0.96 - 1.13) | 1.04 (0.96 - 1.13) |
| HAC (>280g/week) | -- | -- | -- | 4.14 (1.14 - 15.09) | 3.86 (1.06 -14.10) |
| ESR (mm/h) | -- | -- | -- | 1.03 (1.01 - 1.06) | 1.02 (1.00 - 1.05) |
| AST (UI/L) | -- | -- | -- | 1.03 (1.00 - 1.06) | 1.04 (1.00 - 1.07) |
| Heart failure | -- | -- | -- | 5.85 (2.27 - 15.07) | 8.82 (3.29 - 23.64) |
| Stroke | -- | -- | -- | 3.24 (1.04-10.08) | 3.95 (1.28 - 12.25) |
| LogLik ratio test | -- | <0.001 | | <0.001 | |

| | | | | | |
|---|---|---|---|---|---|
| *Risk factors of cardiovascular death were selected based on the elastic net regularization method. Abbreviations: AST, aspartate aminotransferase; B, balance; BMI, body mass index; ESR, erythrocyte sedimentation rate; GP, glycome peak; HAC, heavy alcohol consumption; HR, hazard ratio; ref, reference.* | | | | | |

## Claims

1. *In vitro* method for predicting mortality risk, the method comprising the assessment of the abundance of at least a N-glycome peak selected from the list consisting of: N-glycome peak 16, N-glycome peak 22, N-glycome peak 17, N-glycome peak 23, N-glycome peak 7 and/or N-glycome peak 9 in a biological sample obtained from the subj ect.

2. *In vitro* use of at least a N-glycome peak selected from the list consisting of: N-glycome peak 16, N-glycome peak 22, N-glycome peak 17, N-glycome peak 23, N-glycome peak 7 and/or N-glycome peak 9, or of a kit comprising reagents for assessing the abundance of at least a N-glycome peak selected from the list consisting of: N-glycome peak 16, N-glycome peak 22, N-glycome peak 17, N-glycome peak 23, N-glycome peak 7 and/or N-glycome peak 9, for predicting mortality risk.

3. *In vitro* method, or *in vitro* use, for predicting mortality risk, according to any of the previous claims, wherein an increased abundance of N-glycome peak 16, N-glycome peak 17, N-glycome peak 23, and/or N-glycome peak 7 is an indication of increased mortality risk.

4. *In vitro* method, or *in vitro* use, for predicting mortality risk, according to any of the previous claims, wherein an increased abundance ofN-glycome peak 16 is an indication of all-cause mortality risk, wherein an increased abundance of N-glycome peak 16, N-glycome peak 17 and/or N-glycome peak 23 is an indication of increased cancer mortality risk, and/or wherein an increased abundance of N-glycome peak 7 is an indication of increased cardiovascular mortality risk.

5. *In vitro* method, or *in vitro* use, for predicting mortality risk, according to claims 1 or 2, wherein an increased abundance of N-glycome peak 22 and/or N-glycome peak 9 is an indication of decreased mortality risk.

6. *In vitro* method, or *in vitro* use, for predicting mortality risk, according to claim 5, wherein an increased abundance of N-glycome peak 22 is an indication of decreased all-cause mortality risk or an indication of decreased cancer mortality risk, and/or wherein an increased abundance of N-glycome peak 9 is an indication of decreased cardiovascular mortality risk.

7. *In vitro* method, or *in vitro* use, for predicting all-cause mortality risk, according to any of the previous claims, which comprises assessing the abundance of a combination of N-glycome peaks comprising: N-glycome peak 16 and N-glycome peak 22, wherein an increased abundance of N-glycome peak 16 is an indication of increased all-cause mortality risk, and/or wherein an increased abundance of N-glycome peak 22 is an indication of decreased all-cause mortality risk.

8. *In vitro* method, or *in vitro* use, for predicting cancer mortality risk, according to any of the claims 1 to 6, which comprises assessing the abundance of a combination of N-glycome peaks comprising: N-glycome peak 16, N-glycome peak 17, N-glycome peak 23 and N-glycome peak 22, wherein an increased abundance of N-glycome peak 16, N-glycome peak 17 and/or N-glycome peak 23 is an indication of increased cancer mortality risk, and/or wherein an increased abundance of N-glycome peak 22 is an indication of decreased cancer mortality risk.

9. *In vitro* method, or *in vitro* use, for predicting cardiovascular mortality risk, according to any of the claims 1 to 6, which comprises assessing the abundance of a combination of N-glycome peaks comprising: N-glycome peak 7 and N-glycome peak 9, wherein an increased abundance of N-glycome peak 7 is an indication of increased cardiovascular mortality risk, and/or wherein an increased abundance of N-glycome peak 9 is an indication of decreased cardiovascular mortality risk.

10. *In vitro* method, or *in vitro* use, according to any of the previous claims, wherein the biological sample is selected from blood, plasma or serum.

11. *In vitro* method, or *in vitro* use, according to any of the previous claims, wherein the biological sample is serum.

12. *In vitro* method, or *in vitro* use, according to any of the previous claims, wherein N-glycome peak 16 comprises the glycans: A2[3]BG1S[3]1, A2[3]BG1S[6]1, M7 D1, FA2[6]G1S[3]1, FA2[6]G1S[6]1, M4A1G1S[3]1, M4A1G1S[6]1; N-glycome peak 22 comprises the glycans: FA2G2S[6]1, FA2G2S[3]1, M8 D1 D3; N-glycome peak 17 comprises the glycans: FA2[3]G1S[6]1, FA2[3]G1S[3]1, FA2[6]BG1S[6]1, FA2[6]BG1S[3]1; N-glycome peak 23 comprises the glycans: FA2BG2S[3]1, FA2BG2S[6]1, N-glycome peak 7 comprise the glycan: A2[6]BG1 and N-glycome peak 9 comprise the glycan: FA2[3]G1.

13. *In vitro* method, or *in vitro* use, according to any of the previous claims, wherein N-glycans are separated and quantified using a quantitative analytical technique.

14. *In vitro* method, or *in vitro* use, according to any of the previous claims, wherein the abundance of N-glycome peaks are assessed by chromatography, preferably by ultra-performance liquid chromatography (UPLC), capillary electrophoresis (CE), MALDI-TOF mass spectrometry, liquid chromatography coupled with mass spectrometry (LC-MS).

15. *In vitro* method, or *in vitro* use, according to any of the previous claims, wherein the abundance of N-glycome peaks are assessed by ultra-performance liquid chromatography (UPLC).
